# EUROPEAN PATENT APPLICATION

(11) **EP 2 305 292 A1**
(43) Date of publication of application: **06.04.2011**
(21) Application number: 08808662.4
(22) Date of filing: 01.08.2008
(51) Int. Cl.: A61K 39/00, A61K 9/10, A61K 47/14, A61P 31/00, A61P 37/04

(54) **S/O TYPE TRANSDERMAL IMMUNIZING AGENT**

(71) Applicant: SO Pharmaceutical Corporation, Chuo-ku, Kobe-shi Hyogo 650-0047 (JP); Kyushu University, National University Corporation, Higashi-ku Fukuoka-shi Fukuoka 812-8581 (JP)
(72) Inventor: GOTO, Masahiro, Fukuoka-shi Fukuoka 812-8581 (JP); KAMIYA, Noriho, Fukuoka-shi Fukuoka 812-8581 (JP); HIRATA, Akihiko, Kobe-shi Hyogo 650-0047 (JP); FUJII, Takeru, Kobe-shi Hyogo 650-0047 (JP)
(74) Representative: Gille Hrabal Struck Neidlein Prop Roos
(86) International application number: PCT/JP2008/063910
(87) International publication number: WO 2010/013350

(57) **Abstract**

It is an objective of the present invention to provide a non-invasive transdermal immunizing technology by which inflammation and lump do not appear at the skin unlike conventional transdermal immunizing methods with subcutaneous administration and the development amount of antibody in serum is increased. The S/O type transdermal immunizing agent according to the present invention comprises an antigen-surfactant complex and an oil phase; wherein the antigen is covered with the surfactant in the complex; the complex is in a solid state; and the complex is dissolved or dispersed in the oil phase.

## Description

### TECHNICAL FIELD

The present invention relates to an S/O type transdermal immunizing agent which does not specifically cause dermal inflammation by transdermal immunization and can improve the development of antibody.

### BACKGROUND ART

Dermal tissue plays a role as a physicochemical barrier which nonspecifically prevents the invasion of various infectious organisms such as bacteria and virus. In addition, immune function, by which antibody is developed in a living body, can be specifically improved by subcutaneously-administering antigen such as the part of etiological organism.

However, in transdermal immunization by subcutaneous injection, highly-pure antigen which is aseptically produced is needed, and there are problems of strong side effect, risk such as second infection by injection, and inflammation and lump in the subcutaneously-injected tissue.

As transdermal immunizing methods other than subcutaneous injection using a needle, are disclosed a method in which the stratum corneum at topmost layer of skin is dissolved by some way in Patent Document 1, a method in which the skin is perforated using a minute umbonal needle in Patent Document 2, and a method in which the skin is scratched in Patent Document 3. However, the fact remains that the stratum corneum having a function of barrier is physically invaded in the methods as well as subcutaneous injection except that a needle is not used.

Unlike the above methods, a method in which the toxin derived from pathogenic microorganism is used has been known as a non-invasive transdermal immunizing method in which the skin is not physically invaded by antigen (for example, Patent Documents 4 to 7 and Non-patent Documents 1 to 3). However, the method has a safety problem on the point that the toxin derived from pathogenic microorganism is used.

Meanwhile, an S/O: Solid in Oil type preparation has been known. By the S/O type preparation, the absorbability of mainly enzyme, bioactive peptide and hydrophilic medicine can be improved. The S/O type preparation is produced by dispersing or dissolving the medicine coated with a surfactant in an oil phase such as soybean oil. Depending on the medicine concentration or the like, a transparent S/O type preparation in which the medicine is completely dissolved in the oil phase or a suspension S/O type preparation in which the medicine is dispersed in the oil phase can be obtained.

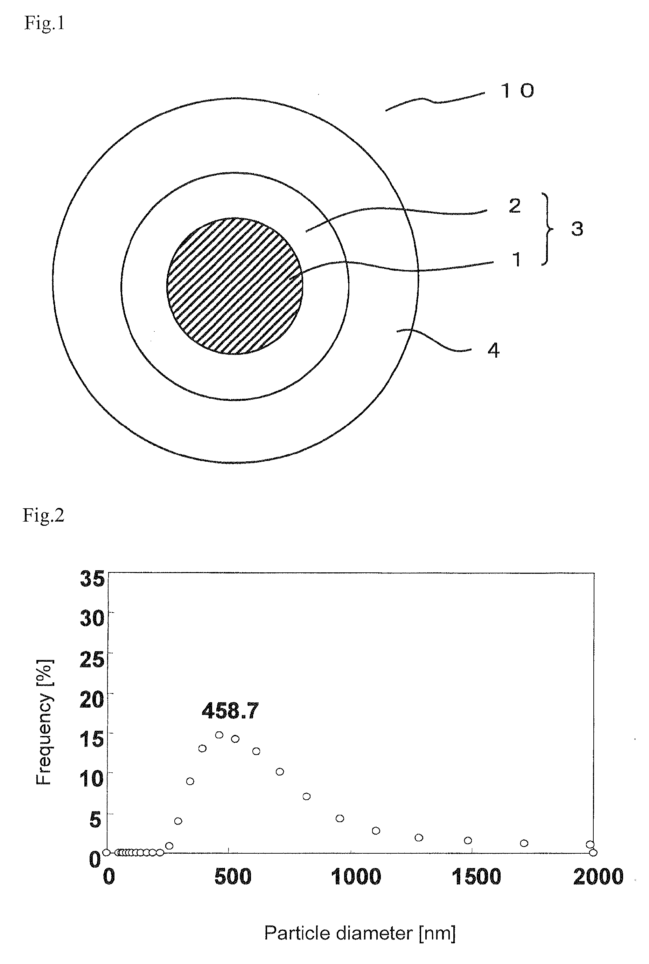

Figure 1 is a view showing a frame format of the structure of an S/O type preparation 10. A medicine 1 is coated with a molecular film of a surfactant 2 to be a solid medicine-containing complex 3, and the solid medicine-containing complex 3 is dispersed in an oil phase 4. Such an S/O type preparation is generally produced by mixing a medicine solution with an organic solvent solution of a surfactant, and lyophilizing the obtained W/O type emulsion in order to obtain a solid medicine-containing complex in which the medicine is coated with the surfactant 2, and dissolving or dispersing the complex in the oil phase. The S/O type preparation has the advantage that (a) the medicine is stabilized against hydrolysis, since the medicine is in a solid state, (b) the preparation is thermally stable, (c) the preparation can be stored for a long time at room temperature, (d) the preparation can be a tape preparation.

In particular, the S/O type preparation from which the leakage of a hydrophilic medicine is prevented could be developed, since a hydrophilic medicine can be dissolved into an oil phase to prevent the leakage of the medicine by using S/O technology.

The present inventor developed the S/O preparation described in, for example, Patent Documents 8 to 10. Among them, Patent Document 8 discloses the S/O type preparation containing peptide such as insulin or camptothecin derivative such as irinotecan hydrochloride, and the S/O/W type preparation obtained by dispersing the S/O type preparation into an aqueous phase. In addition, Patent Document 9 discloses the S/O technology obtained by covering the mixture containing a hydrophilic medicine and a hydrophilic medicine-leakage-suppressive protein and/or a medicine-leakage-suppressive polysaccharide as a stabilizing agent with a surfactant; and it is described in Patent Document 9 that the leakage of a low-molecular medicine is significantly reduced in a strong acidic environment and the release of the low-molecule medicine is accelerated when the preparation comes into contact with the enteric canal or the like in a weak acidic to neutral environment. This S/O technology is very useful for particularly the medicine which has a problem of the serious disturbance of stomach mucosa, such as gastric ulcer. Such a medicine is exemplified by water-soluble nonsteroidal antiphlogistic analgetic such as diclofenac sodium (DFNa) and aspirin. The nonsteroidal antiphlogistic analgetic is hereinafter referred as NSAIDs. Furthermore, Patent Document 10 discloses the S/O type external preparation which is capable of improving skin permeability of hydrophilic medicines such as NSAIDs.
Patent Document 1: JP 2002-504522 T
Patent Document 2: JP 2005-334594 A
Patent Document 3: JP 2005-511248 T
Patent Document 4: JP 2005-179301 A
Patent Document 5: JP 2004-536804 T
Patent Document 6: JP 2005-504002 T
Patent Document 7: JP 2006-503072 T
Patent Document 8: JP 2004-43355 A
Patent Document 9: WO 2005/094789
Patent Document 10: WO 2006/025583
Non-patent Document 1: F. Mawas, et al., The Journal of Infection Disease, 190; 1177-1182, 2004
Non-patent Document 2: M.Guebre-Xabier, et al., Journal of Virology, 78: 7640-7618, 2004
Non-patent Document 3: G.M. Glenn, et al., Nature Med. 6(12): 1403-1406, 2000

### DISCLOSURE OF THE INVENTION

### PROBLEMS TO BE SOLVED BY THE INVENTION

The objective of the present invention is to provide non-invasive transdermal immunizing technology by which inflammation and lump do not appear at the skin such as stratum corneum unlike conventional transdermal immunizing methods with subcutaneous administration and the development amount of antibody in serum is increased. More specifically, the objective of the present invention is to provide a novel transdermal immunizing technology by which stratum corneum playing a role as physicochemical barrier on the outermost layer of the skin tissue is not destroyed by physical invasion such as dissolution, punching and paratripsis, unlike the documents described the above BACKGROUND ART; which is very safe non-invasive transdermal immunizing method in which toxin derived from pathogenic microorganism is not used; which is excellent in the ability of antibody development; and by which transdermal immunization can be effectively carried out.

### MEANS FOR SOLVING THE PROBLEMS

The S/O type transdermal immunizing agent according to the present invention, which can solve the above-mentioned problems, is characterized in comprising an antigen-surfactant complex and an oil phase; wherein the antigen is covered with the surfactant in the complex; the complex is in a solid state; and the complex is dissolved or dispersed in the oil phase.

The antigen is preferably is a protein or peptide.

The antigen is preferably is an unchanged or inactivated bacterium and/or virus.

The antigen is preferably is derived from an animal or plant.

The HLB value of the surfactant is preferably not more than 10.

The oil phase is preferably selected from a group consisting of a plant oil, an animal oil, a neutral lipid and a long-chain fatty acid ester.

The present invention also relates to the S/O/W type transdermal immunizing agent, wherein the S/O type transdermal immunizing agent is dispersed in an aqueous phase.

### EFFECT OF THE INVENTION

According to the present invention, not only the development amount of antibody can be increased similarly to conventional transdermal immunizing methods with subcutaneous injection, but also all of problems by subcutaneous injection, such as inflammation of the skin tissue, the accident by needle, dissolution and destruction of stratum corneum playing a role as physicochemical barrier, can be solved. The transdermal immunizing agent according to the present invention is therefore very useful as a non-invasive immunizing agent or vaccine with high-safety.

The transdermal immunizing agent according to the present invention is useful not only as protection means or exclusion means of pathogenic bacteria and virus from living body, but also for a treatment method of producing immunological tolerance by transdermally administering a small amount of antigen in several batches.

### BRIEF DESCRIPTION OF THE DRAWINGS

Fig.1 is a view showing a frame format of the structure of an S/O type preparation.
Fig.2 shows the particle size distribution of the preparation according to the present invention.
Fig.3 shows the time-dependent change of the antibody amount in each group in Test example 1.
Fig.4A shows a photograph of the pathological tissue magnified 80 times when the present invention agent was applied on the earflap.
Fig.4B shows a photograph of the pathological tissue magnified 400 times when the present invention agent was applied on the earflap.
Fig.5A shows a photograph of the pathological tissue magnified 80 times when the present invention agent was applied on the back.
Fig.5B shows a photograph of the pathological tissue magnified 400 times when the present invention agent was applied on the back.
Fig. 6A shows a photograph of the pathological tissue magnified 80 times when comparative agent 1 containing FCA was administered.
Fig.6B shows a photograph of the pathological tissue magnified 200 times when comparative agent 1 containing FCA was administered.
Fig. 6C shows a photograph of the pathological tissue magnified 400 times when comparative agent 1 containing FCA was administered.
Fig. 7A shows a photograph of the pathological tissue magnified 80 times when comparative agent 2 containing no FCA was administered.
Fig.7B shows a photograph of the pathological tissue magnified 200 times when comparative agent 2 containing no FCA was administered.
Fig. 7C shows a photograph of the pathological tissue magnified 400 times when comparative agent 2 containing no FCA was administered.

### EXPLANATIONS OF LETTERS OR MUMERALS

1 : medicine
2 : surfactant
3 : medicine-containing complex (solid phase)
4 : oil material (oil phase)
10 : S/O type preparation

### BEST MODE FOR CARRYING OUT THE INVENTION

The transdermal immunizing agent according to the present invention has the greatest characteristics in that antigen is included in S/O preparation by S/O technology described in the above Patent Document 7. An antigen typified by a hydrophilic polymer such as peptide naturally has very low skin permeability; on the other hand, according to the present invention, the skin permeability of antigen can be increased, inflammation of the skin is remarkably reduced, and antibody is sufficiently developed, since antigen is dissolved in oil material by S/O technology. More specifically, as Examples described later, S/O transdermal immunizing agent (S/O antigen) was produced by using a lysozyme derived from a hen egg albumen as an antigen, covering the hen lysozyme antigen with a surfactant to obtain a W/O type emulsion and hydrophobize the antigen, dispersing the emulsion in a oil phase (dispersion medium) such as plant oil. The S/O transdermal immunizing agent was applied on the earflap or back of rabbit for transdermal immunization. As a result, it was demonstrated that inflammation and lump were not observed under the skin, compared to conventional subcutaneous immunizing method, and the antibody specific hen lysozyme was developed in serum.

The S/O type transdermal immunizing agent according to the present invention contains a solid antigen-surfactant complex (A) and oil phase (B), an antigen (A-1) is covered with a surfactant (A-2) in the complex, and the solid antigen-surfactant complex (A) is dissolved or dispersed in the oil phase (B). The antigen-surfactant complex (A) preferably contains a hydrophilic protein and/or a hydrophilic polysaccharide (A-3) as a stabilizing agent.

The present invention agent is substantially the same as S/O type agent described in the above-mentioned Patent Document 7 except that antigen was contained in place of medicine in the S/O type preparation described in the above-mentioned Patent Document 8. Hereinafter, the constituent features are specifically explained.

### (A) Antigen-surfactant complex

The antigen-surfactant complex contains an antigen and surfactant as main component. Hereinafter, the antigen-surfactant complex is sometimes referred as "antigen-containing complex". The antigen interacts with the hydrophilic part of the surfactant, to be covered. The antigen-surfactant complex may consist of the antigen and surfactant only so that the particle size may not be too large; or may contain a stabilizing agent or adjuvant (auxiliary agent) used for an antibody-development-enhancing agent or other preparation component (addition ingredient) acceptable for pharmaceutical preparation, as described later.

### (A-1) Antigen

The antigen used in the present invention is particularly not limited as long as it stimulates an immune response at the skin and induces immunogenicity to develop antibody. Antigen, vaccine, immunogenic compound and the like used for conventional transdermal immunization are used as the antigen. Specifically, bioactive protein and peptide such as lysozyme derived from hen egg white and antigenic domain peptide of cedar pollen allergy, haptened substance having protein or peptide, unchanged or inactivated bacteria or virus and partial degradation product thereof, inactivated cancer cell and partial degradation product thereof, and nucleic acid are exemplified. In addition, the substance having no antigenicity, such as carbohydrate, lipid and inorganic compound, may be antigenic by means such as haptenization, to be used as the antigen. The antigen may be derived from an animal or plant. For example, the antigen may be ones for poultry, domestic animal and pet animal; ones for bovine, hen and hog; and ones derived from insect such as ticks. The antigen may be also derived from food.

### (A-2) Surfactant

The surfactant is not particularly limited insofar as it is pharmaceutically acceptable, and the examples thereof include a nonionic surfactant, an anionic surfactant, a cationic surfactant, an amphoteric surfactant, and a bile salt.

The examples of nonionic surfactant include polyglycerol esters condensation ricinoleic acid, decaglycerin fatty acid ester, glycerol fatty acid ester, polyglycerol fatty acid ester, polyoxy ethylene glycerol fatty acid ester, sorbitan fatty acid ester, polyoxyethylene sorbitol fatty acid ester, polyoxy ethylene castor oil or hydrogenated castor oil, and sucrose fatty acid ester (sucrose stearic acid ester, sucrose palmitic acid ester, sucrose myristic acid ester, sucrose oleic acid ester, sucrose lauric acid ester, sucrose erucic acid ester, and sucrose mixed fatty acid ester). One of them may be selected or more than one may be selected and combined for use.

As the nonionic surfactant, an ester compound produced by using unsaturated fatty acid such as erucic acid or oleic acid as an ingredient is preferred, and more preferred are sucrose erucic acid ester, sucrose oleic acid ester, sucrose mixed fatty acid ester. Alternatively, one, or two or more selected from a group consisting of glycerol fatty acid ester, polyglycerol fatty acid ester, polyoxyethylene glycerol fatty acid ester, sorbitan fatty acid ester, sucrose fatty acid ester, polyoxy ethylene sorbitol fatty acid ester and polyoxy ethylene castor oil or hydrogenated castor oil may be used.

As the surfactant, a surfactant of high hydrophobicity having a HLB value of 10 or less is preferably used, since such a surfactant easily dissolves or disperses the antigen-containing complex in an oil phase.

In the antigen-containing complex (A), hydrophilic medicine (A-1) is covered with surfactant (A-2) as mentioned above. In addition, the complex may further contain a hydrophilic protein and/or a hydrophilic polysaccharide (A-3) as a stabilizing agent.

### (A-3) Hydrophilic protein and/or hydrophilic polysaccharide as a stabilizing agent

The stabilizing agent used in the present invention is covered with surfactant as well as antigen; as a result, improves the stability of the antigen-containing complex. In particular, the stabilizing agent has the effect of preventing the leakage of the antigen from the antigen-containing complex of the transdermal agent. The kind of the stabilizing agent, i.e. the hydrophilic protein and/or hydrophilic polysaccharide, is not limited as long as it is acceptable as the component of pharmaceutical preparation. However, the protein having a number average molecular weight of not less than 10,000 is preferably used for effective suppressive property of medicine leakage.

The hydrophilic protein is exemplified by serum albumin (molecular weight: about 67,000), ovalbumin (molecular weight: about 45,000), casein (molecular weight: about 19,000 or more), lysozyme (molecular weight: about 14,000 or more), and lipase (molecular weight: about 45,000). One of these may be selected or more than one may be selected and combined for use. Preferably, one or more than one selected from a group consisting of serum albumin, ovalbumin and casein may be used.

The hydrophilic polysaccharide is exemplified by maltose, sucrose, lactose, trehalose, cellobiose, pullulan, LM pectin, HM pectin, hydroxypropylmethyl cellulose phthalate, heparin, alginic acid and carboxymethyl cellulose; and one of them may be selected or more than one may be selected and combined for use. Preferably, one or more than one selected from a group consisting of sucrose, trehalose, pullulan, LM pectin, HM pectin and hydroxypropylmethyl cellulose phthalate may be used.

In the antigen-containing complex, the weight ratio of the stabilizing agent relative to the antigen is preferably in the range of not less than 0.01 and not more than 100. If the ratio is less than 0.01, the suppressive property of medicine leakage may not be sufficiently achieved, while if the ratio is more than 100, the amount of medicine occupied in the complex becomes small so that the original effects of the medicine may not be exerted. More preferably, the ratio is within the range of not less than 0.1 and not more than 10, even more preferably not less than 0.5 and not more than 5.

The weight ratio of the surfactant relative to the combined amount of the antigen and the stabilizing agent is preferably within the range of not less than 0.5 and not more than 100, more preferably not less than 1 and not more than 50, even more preferably not less than 2 and not more than 25.

### (A-4) Other component

The antigen-containing complex (A) of the present invention has the structure in which antigen (A-1) is covered with surfactant (A-2) as described above. In addition, the complex (A) may further contain adjuvant for improving the production ability of antibody. Adjuvant plays a role as regulatory factor for assisting the induction of immunological response, and is usually administered with antigen. In the present invention, adjuvant is an optional component and not an essential component. It is experimentally demonstrated by Examples described later that antibody development can be improved without the addition of adjuvant by using the S/O type transdermal immunizing agent according to the present invention.

The adjuvant used in the present invention is not limited as long as it is usually used for subcutaneous injection. The adjuvant is specifically exemplified by the mineral oil developed by Freund, the mixture of surface-active agent and tuberculous organism killed by heating (FCA: Freund's complete adjuvant), heat-labile toxin such as cholera toxin and toxigenic Escherichia coli. In addition, the adjuvant is preferably exemplified by mutant type detoxified cholera toxin and mutant type detoxified heat-labile toxin which is detoxified by removing diarrhetic ADP-ribocyltransferase activity and have adjuvant activity.

As the production method of the S/O type transdermal immunizing agent according to the present invention with the addition of adjuvant, (a) adjuvant may be added with antigen in order to obtain antigen-adjuvant-surfactant complex by the method described later, and then the complex may be dissolved or dispersed in an oil material, or (b) adjuvant-surfactant complex may be produced as the production method of antigen-surfactant complex, and then both adjuvant-surfactant complex and antigen-surfactant complex are dissolved or dispersed in an oil material by the method described later.

### (B) Oil phase

The S/O type transdermal immunizing agent of the present invention is a solution or a suspension wherein the antigen-containing complex as previously explained is dissolved or dispersed in an oil phase. It is dependent on the type and amount of surfactant and oil phase, and with or without treatment of sonication and the like whether the agent becomes a solution or a suspension.

The oil phase used in the present invention is not particularly limited insofar as it is pharmaceutically acceptable, and examples thereof include plant oil, animal oil, neutral lipid such as mono-substituted, di-substituted or tri-substituted glyceride, long-chain fatty acid ester, synthetic oil, and sterol derivatives.

The specific examples include plant oils such as soybean oil, cottonseed oil, rape seed oil, sesame oil, corn oil, peanut oil, safflower oil, sunflower oil, olive oil, rape seed oil, perilla oil, fennel oil, cacao seed oil, cinnamon oil, mentha oil and bergamot oil; animal oils such as beef fat, pork oil and fish oil; neutral lipids such as medium-chain fatty acid triglyceride, triolein, trilinolein, tripalmitin, tristearin, trimyristin and triarachidonin; synthetic lipids such as azone; sterol derivatives such as cholesteryloleate, cholesteryl linoleate, cholesteryl myristate, cholesteryl palmitate and cholesteryl arachidate; long chain fatty acid esters such as isopropyl myristate, octyldodecyl myristate, cetyl myristate, ethyl oleate, ethyl linoleate, isopropyl linoleate, isopropyl palmitate and butyl stearate; lactate esters such as ethyl lactate and cetyl lactate; a multivalent carboxylic acid ester such as triethyl citrate, diisopropyl adipate, diethyl sebacate and diisopropyl sebacate; other carboxylic acid esters such as cetyl 2-ethylhexanoate; hydrocarbons such as petrolatum, paraffin and squalane; and silicones. One of them may be selected or more than one may be selected and combined for use. Preferably, one or more than one selected from a group consisting of soybean oil, sesame oil, olive oil, safflower oil, sunflower oil, rape seed oil and perilla oil is used. Particularly it is preferred to use triglyceride or cooking oils based on the same, and practically preferred is soybean oil, especially highly-purified soybean oil. In addition, preferably a neutral lipid or long chain fatty acid ester, more preferably a long chain fatty acid ester, even more preferably an isopropyl myristate may be used.

The ratio of the oil phase contained in the S/O type transdermal agent of the present invention differs depending on the type of oil component and other constituting components and the like, and is preferably within the range of not less than 50 w/v % and not more than 99.5 w/v %, more preferably not less than 60 w/v % and not more than 90 w/v%.

The S/O/W type agent of the present invention is obtained by dispersing the S/O type transdermal immunizing agent in an aqueous phase. The agent can be easily administered and has a property of high convenience.
The kind of the aqueous phase is not particularly limited as long as it is acceptable for pharmaceutical preparation, and, for example, pure water, purified water, distilled water, saline and buffer solution may be used.

Next, the method for producing the S/O type transdermal immunizing agent of the present invention is explained.

The method for producing the agent of the present invention comprises the steps of (1) mixing an antigen and a surfactant, and an adjuvant and organic solvent solution containing a stabilizing agent if necessary, to obtain a W/O type emulsion, (2) drying the W/O type emulsion to obtain an antigen-containing complex, and (3) dissolving or dispersing the antigen-containing complex in an oil phase.

### (1) Step for preparing W/O type emulsion

In the step, first, the aqueous solution of antigen is produced. If necessary, adjuvant and/or stabilizing agent is also added thereto. In addition, additional ingredient acceptable for pharmaceutical preparation may be added. The water to be used in the step is exemplified by pure water, purified water, distilled water, saline and buffer solution. A small amount of water miscible organic solvent such as ethanol may be added as necessary. However, caution should be taken as it becomes difficult to form an emulsion if too much alcohol and the like are added.

The concentration of the antigen, or the total concentration if adjuvant and/or stabilizing agent is further added, is not particularly limited as long as the component can be substantively completely dissolved, and for example, the concentration may be about not less than 0.1 mg/mL and not more than 30 mg/mL.

Separately, an organic solvent solution of the surfactant is prepared. The organic solvent is not particularly limited insofar as it is able to dissolve the surfactant and removable by diffusion in the subsequent step. The examples of such solvent include alcohols such as methanol and ethanol; aliphatic hydrocarbons such as hexane; and aromatic hydrocarbons such as toluene; ester solvents such as ethyl acetate; halogenated hydrocarbons such as methylene chloride. The concentration is not particularly limited, and may be, for example, about not less than 1% by mass and not more than 10% by mass.

Next, a W/O type emulsion is prepared by mixing the aqueous solution of the antigen which solution may be contain adjuvant and/or stabilizing agent, with the organic solvent solution of the surfactant according to a conventional method. For example, high speed stirring using homogenizer, stirring by a stirrer such as propeller mixer or disper, and additionally irradiation of ultrasonic wave may be employed. Alternatively, the W/O type emulsion may be prepared by membrane emulsification using porous membrane. As the porous membrane, for example, commercially available hydrophobic shirasu porous glass membrane such as SPG manufactured by SPG Technology Co., Ltd is preferably used. The porous membrane has the advantage that the dispersed particles of antigen-containing complex having the diameter corresponding to the micropore diameter of the porous glass membrane can be obtained.

### (2) Drying step

In the step, the W/O type emulsion obtained in the step (1) described above is dried to obtain the antigen-containing complex. The drying method is not particularly limited. A method such as lyophilization and drying under reduced pressure condition may be employed, and lyophilization is preferable. As to the specific conditions, conventional method is employed. In this step, it is preferable to substantially-completely remove the water and the organic solvent. Moisture may cause a hydrolysis of protein or antigen, and the organic solvent, needless to say, may adversely affect on a living body. More specifically, the moisture content should be about not more than 1% in the measurement by the Karl Fischer method.

### (3) Dispersing step

In the step, the antigen-containing complex obtained in the step (2) described above is dissolved or dispersed in the oil phase, to thereby produce the S/O type agent. More specifically, high speed stirring using homogenizer, stirring by a stirrer such as propeller mixer or disper, and additionally irradiation of ultrasonic wave may be employed.

The amount of the oil phase used in this step is, for example, about not less than 1 mL and not more than 10 mL per 1 g of the antigen-containing complex, although it depends on the kind and affinity of the surfactant and the oil phase or the like.

The obtained S/O type agent may further be dispersed in an aqueous phase according to a conventional method to give an S/O/W type agent.

The above mentioned solution or suspension may be directly applied on body part such as inside of the arm, of which part skin is thin and which is easily applied; however, preferably another additional ingredient is added thereto to make it a pharmaceutical preparation. Examples of another additional ingredient include diluent (for example, sugars such as sucrose; a starch derivative such as dextrin; cellulose derivatives such as carmellose sodium; a water-soluble polymer such as xanthan gum), colorant, binding agent (such as above-described diluent and macrogol), emulsifying agent, thickener, moisturizer such as glycerin, stabilizer (for example, a p-hydroxybenzoic acid ester such as methyl paraben and propylparaben; an alcohol such as chlorobutanol, benzylalcohol, and phenylethylalcohol; benzalkonium chloride; phenols such as phenol and cresol; thimerosal; acetic anhydride; sorbic acid, and the like), preservative, solvent (such as water, ethanol, glycerin, and a higher alcohol such as oleyl alcohol), solubilizing agent, suspending agent (such as carmellose sodium), buffering agent, pH adjuster, base (such as polyethylene glycol, crotamiton, diethyl sebacate and petrolatum), hydrocarbons such as petrolatum and microcry stalline wax, esters such as jojoba oil and spermaceti wax, triglycerides such as beef tallow and olive oil, absorption promoter such as menthol, tonicity agent, organic solvent, and surfactant. The additional ingredient may be added at an ordinary amount.

The above mentioned solution or suspension may be blended with the other additional ingredient by a conventional method according to the form of each drug preparation, to obtain an ointment, a lotion agent, an aerosol agent, a plaster, an aqueous cataplasms, a cream, an ointment, a gel, a plaster, a reservoir type adhesive skin patch, a matrix type adhesive skin patch, and a tape. The present invention is not limited to the above forms.

The dosage amount of the transdermal agent of the present invention may be adjusted according to the type and the amount of the antigen to be blended, and the age and the symptom of the patient, and the like. For example, the transdermal immunizing agent having only antigen and no adjuvant is preferably applied, in general, in the range of about not less than 100 µg and not more than 2000 µg, more preferably not less than 100 µg and not more than 500 µg for each administration for several weeks to several months at least two times to one adult human. The transdermal immunizing agent having both of antigen and adjuvant is preferably applied, in general, in the range of about not less than 50 µg and not more than 500 µg, more preferably not les than 50 µg and not more than 200 µg for each administration for several weeks to several months at least two times to one adult human.

### EXAMPLES

Hereinafter, the present invention is described in detail with reference to examples and test examples; however, it is not intended that the present invention be limited to the illustrated examples.

It is experimentally demonstrated as below that using HEL: Lysozyme from hen egg white as a model antigen for S/O transdermal immunization, the transdermal immunizing method using the S/O transdermal immunizing agent containing HEL does not cause inflammation on the skin unlike conventional subcutaneous immunizing methods and is excellent to antibody development ability.

### Production of the present invention agent

To the HEL solution obtained by dissolving HEL (2.0 mg) in a phosphate buffer saline (PBS, pH 7.4) (2 mL), a 5.0 wt% cyclohexane solution (4.0 mL) of sucrose erucic acid ester (ER-290 manufactured by Mitsubishi-Kagaku Foods Corporation, erucic acid: 90 wt%, HLB: 2) (4.0 mL) was added. The mixture was stirred with high speed at 26,000 rpm for 2 minutes using a homogenizer in order to prepare a W/O type emulsion. The emulsion was lyophilized for a night and day in order to a surfactant-HEL complex.

Isopropyl myristate (4 mL) was added to the complex, and the complex was dispersed in order to obtain an S/O type complex suspension according to the present invention. The suspension contained HEL at a concentration of 0.5 mg/mL.

Fig.2 shows the particle size distribution (frequency distribution) of the HEL-containing complex obtained by the above method. The average particle diameter of the HEL-containing complex was measured by laser scattering method using a Zetasizer nano ZS manufactured by Sysmex Corporation; as a result, the average diameter was 458.7 nm.

### Production of a comparative agent

As positive controls, were prepared comparative agent 1 obtained by adding equal amount of HEL antigen and Freund' s complete adjuvant (FCA) to be emulsified, and comparative agent 2 containing HEL antigen only.

Specifically, to a HEL-containing solution obtained by dissolving HEL (2.0 mg) in phosphate buffer saline (PBS, pH 7.4) (2 mL), equal amount of neat FCA liquid (2 mL) was added to be emulsified, in order to prepare comparative agent 1. In addition, comparative agent 2 was prepared similarly to comparative agent 1 except that FCA was not added.

The following experiments were carried out using thus obtained agent of present invention and comparative agents 1 and 2, and the effects on antibody development amount and administered part were compared by the observation with naked eyes and histopathological work-up.

### Test example 1: Comparison of antibody development amount

Four male rabbits (New Zealand White) having a weight of about 2 kg were prepared, and divided into two for positive control group (for comparative agents 1 and 2) and two for experimental group (for the present invention agent). The administration was carried out as follows.

To the positive control group
The hair on the back of two rabbits of the positive control group was shaved by a shaver, and 1 mL per one rabbit of comparative agent 1 containing adjuvant or comparative agent 2 containing no adjuvant was respectively subcutaneously-injected.

To the experimental group
The hair on the back of one rabbit of two for experimental group was shaved by a shaver similarly to the above, and 1 mL of the present invention agent was applied using a brush for paint in the range within about 10 centimeters square. In addition, 1 mL of the present invention agent was applied on the navicular part of the one earflap of the other rabbit.

To both of the experimental group and positive control group, the same administration was carried out on the 14th day (the first immunity), 28th day (the second immunity), 42nd day (the third immunity) and 59th day (the fourth immunity). Before each of the administration of the first to fourth immunity, blood sample was obtained from the outer vein of the ear, and the anti-HEL antibody amount in the obtained sample was measured by ELISA method. In the case of the group applied with the present invention agent on the earflap, sample was obtained from another ear without application. The details of ELISA method was shown as the following (1) to (5), and the cleaning operation in the each step was sufficiently carried out using a phosphate buffer saline (PBS), PBST (a mixture of PBS and Tween 20 as a hydrophilic surfactant) or 1M NaCl. The measurement was 3 times in total.

(1) A HEL solution in a phosphate buffer saline (PBS, pH 7.4) containing 5 mg of protein was added on a 96-hole microplate at a dose of 200 µL/well and the microplate was still laid at 4°C overnight so that HEL was adsorbed on the microplate.

(2) A PBS solution of BSA: Bovine Serum Albumin, containing 5.0 mg/mL of BSA, was added on the microplate adsorbed with HEL at a dose of 200 µL/well as a blocking agent to prevent nonspecific adsorption, and a blocking operation was carried out at 37°C for 2 hours.

(3) The sample was added at a dose of 200 µL/well to induce antigen-antibody reaction between HEL adsorbed on the microplate and the anti-HEL antibody in the sample at 37°C for 2 hours.

(4) After removing the blocking agent, the microplate was carefully rinsed with PBS containing 0.2% of Tween 80, and a solution obtained by diluting anti-rabbit IgG antibody which was derived from goat and labeled with alkaline phosphatase (AP) with PBS containing 0.1% BSA 5,000 times was added thereto, in order to sufficiently-induce antigen-antibody reaction at 37°C for 2 hours.

(5) After the free anti-rabbit IgG antibody which was derived from goat and labeled with AP was carefully removed by cleaning operation, 1.0 mM p-nitrophenylphosphoric acid dissolved in 1M Tris-HCl buffer of pH 7.5 was added on the microplate at a dose of 200 µL/well, and the amount of anti-HEL antibody in the rabbit serum was obtained by measuring the absorbance at λ = 410 nm.

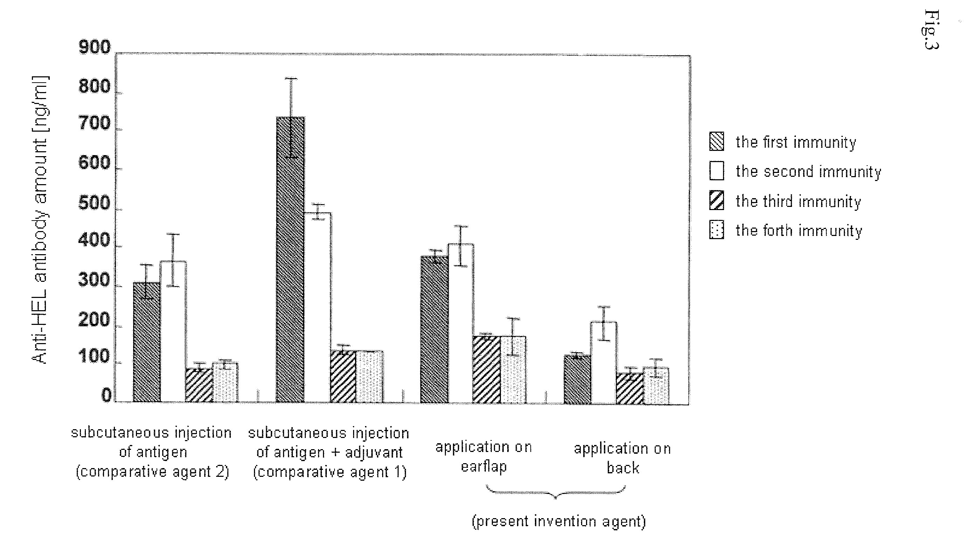

The results are collectively shown in Fig.3. In Fig.3, the results of the positive control group and experimental group are collectively demonstrated. Starting from the left, the results of comparative agent 2 subcutaneously-injected and having no adjuvant, comparative agent 1 subcutaneously-injected and having adjuvant, the present invention agent applied on the earflap, and the present invention agent applied on the back are demonstrated in order.

From Fig.3, the following conclusion can be derived.

First, it was observed that the amount of anti-HEL antibody was increased at the first immunity and the second immunity with transcutaneous immunization by subcutaneously-injecting comparative agents 1 and 2 as the conventional way. In particular, a remarkable increase of antibody amount was observed at the first immunity in the case of comparative agent 1 containing adjuvant.

In addition, in the both cases of the S/O present invention agents applied on the earflap and the back, an increase of antibody was observed at the first immunity and the second immunity. In particular, a remarkable increase of antibody amount was observed in the case where the agent was applied on the earflap, of which skin tissue is thin and skin permeability is high, compared with the case where the agent was applied on the back, of which skin tissue is thick and skin permeability is low; and the degree was approximately the same as the case of comparative agent 2.

### Test example 2: Observation of stimulus property at the administered part by naked eyes

The administered part of the rabbits in the experimental group and positive control group was observed by naked eyes at the first immunity.

As a result, in the cases where comparative agent 1 and comparative agent 2 were subcutaneously-inj ected at the rabbit back, an apparent lump was generated at the first immunity. In particular, in the case where comparative agent 1 containing antigen and adjuvant was subcutaneously-injected, the degree of generation of lump was remarkable (the result is not shown by figure). On the other hand, in the case where the present invention agent was applied not only on the back but also on the navicular part of the earflap, which is sensitive to stimulation, the engorgement of a blood vessel, hyperemia, cutaneous stimulation and the like were not observed at all.

### Test example 3: Histopathological work-up

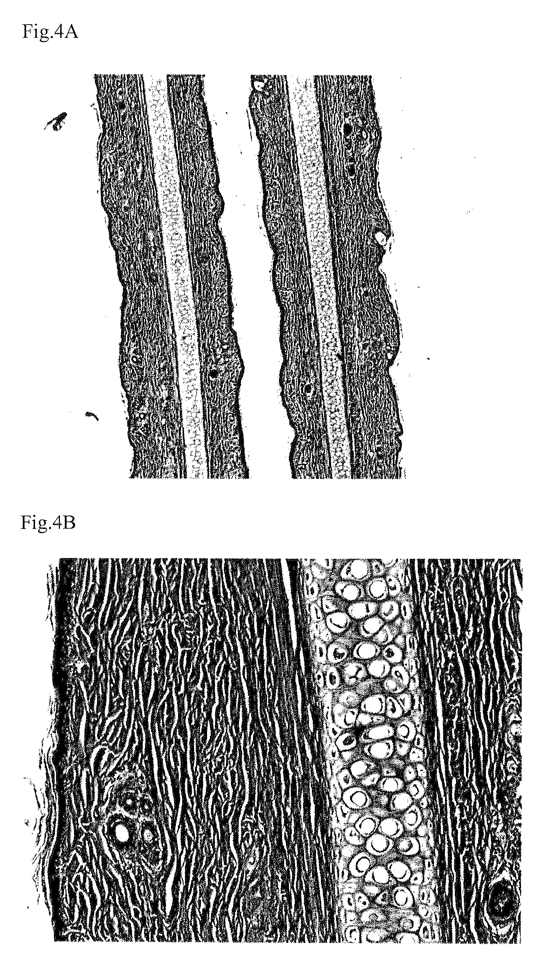

In this test, the skin of the each rabbit 66 days after the administration was stained with hematoxylin-eosin (HE) staining in order to prepare pathological specimen, and then the specimen were observed by an optical microscope at 80-, 200- or 400-fold magnification in order to check inflammation of the skin. Figs.4 to 7 show photographs of the pathological tissue when the present invention agent was applied on the earflap or back, or comparative agent 1 or 2 was injected.

First, the result when the present invention agent was applied was considered.

Fig.4 shows a photograph of the pathological tissue when the present invention agent was applied on the earflap, and Fig.4A is a 80-fold magnified photograph and Fig.4B is a 400-fold magnified photograph. As shown in Figs.4A and 4B, there is no inflammation on the skin of the applied earflap at all, and the subcutaneous tissue containing healthy epidermis, dermis and fat was observed.

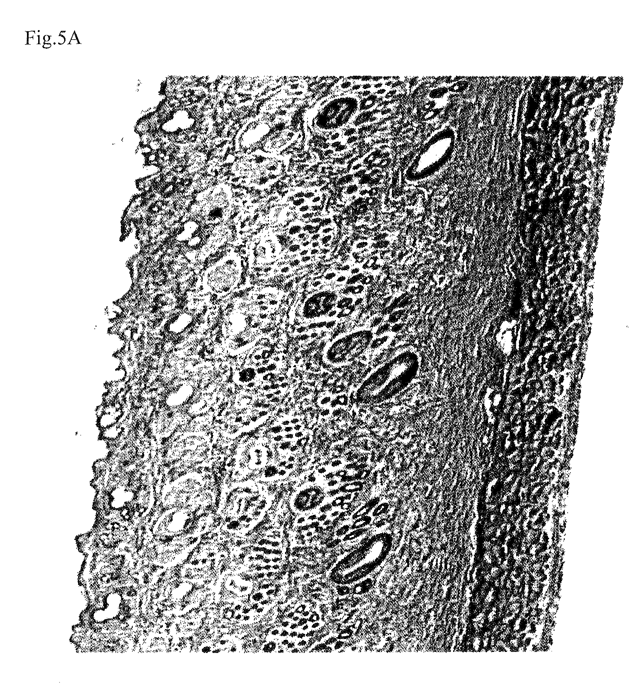

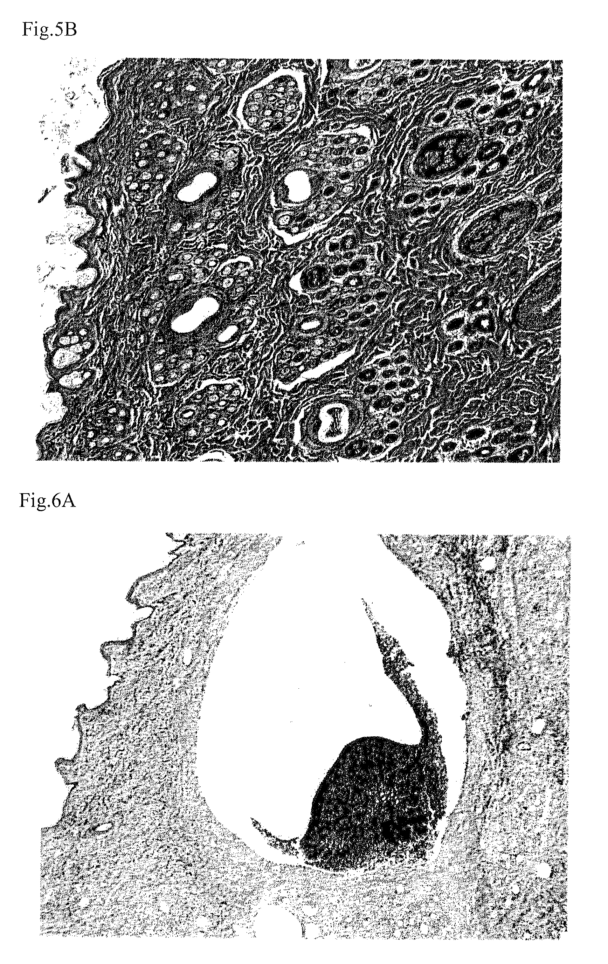

Fig.5 shows a photograph of the pathological tissue when the present invention agent was applied on the back, and Fig.5A is a 80-fold magnified photograph and Fig.5B is a 400-fold magnified photograph. As shown in Figs.5A and 5B, there is no inflammation on the skin of the applied back at all, and the subcutaneous tissue containing healthy epidermis, dermis and fat was observed.
As mentioned above and shown in Fig.4 and Fig.5, there is no inflammation on the skin to be observed in the both cases where transdermal immunization was carried out by applying the present invention agent on the earflap and back.

Next, the result when the comparative agent was administered was considered.

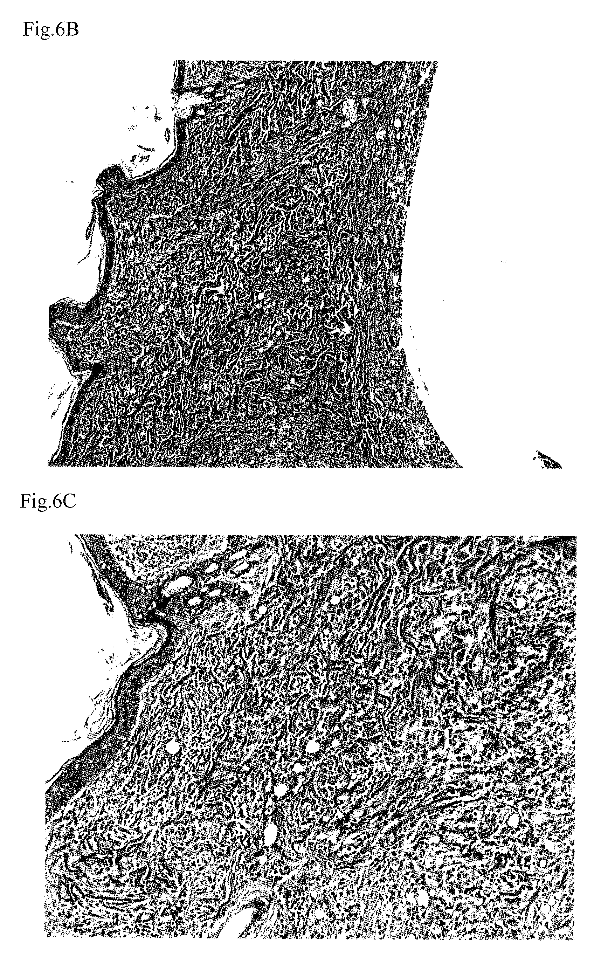

Fig.6 shows a photograph of the pathological tissue when the comparative agent 1 containing FCA was used, and Fig. 6A is a 80-fold magnified photograph, Fig.6B is a 200-fold magnified photograph and Fig. 6C is a 400-fold magnified photograph. As shown in Figs. , large cyst was observed at dermic layer (refer to Fig.6A), and necrotic tissue was observed around the large cyst (refer to Fig.6B). In addition, as shown in Fig.6C, inflammation was observed in a wide range from the dermic layer to hypodermal tissue, and they were thickened. Clusters of macrophage which swelled by containing large vacancy and foamy cell cytoplasm were observed, and cellular infiltration was observed between the clusters.

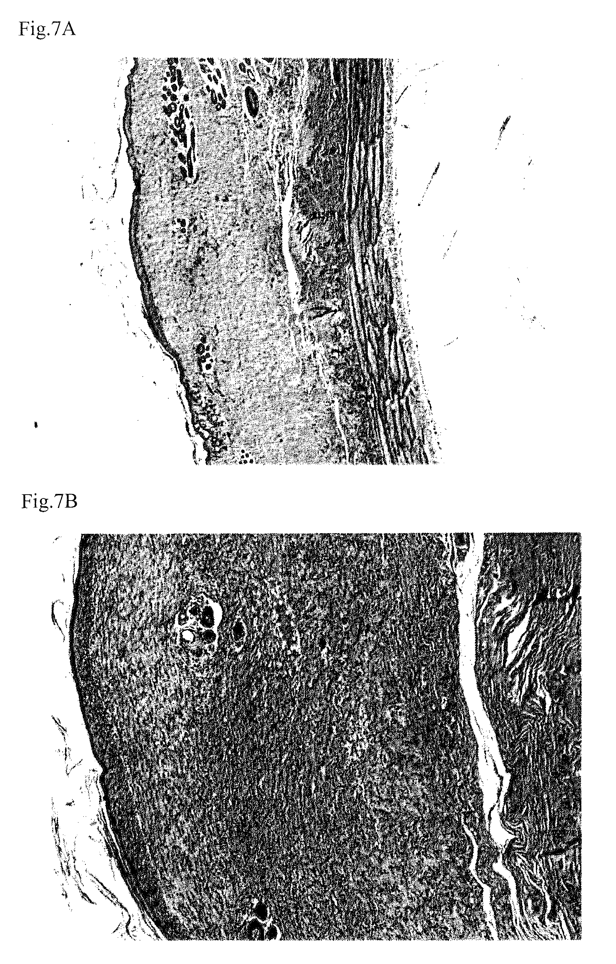

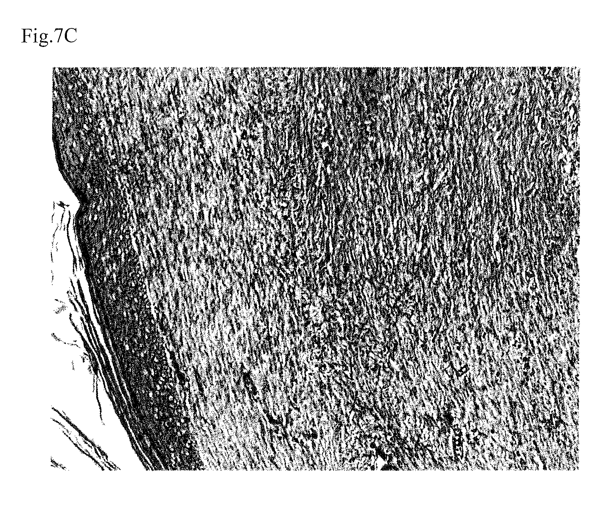

Fig.7 shows a photograph of the pathological tissue when the comparative agent 2 containing no FCA was used, and Fig.7A is a 80-fold magnified photograph, Fig.7B is a 200-fold magnified photograph and Fig. 7C is a 400-fold magnified photograph. As shown in Figs., mild thickness in the part of the emipermis was observed (on the far left of Figs.7A, 7B and 7C), and weak fibroblast cells with mild degree of cell infiltration were densely distributed in the dermic layer therebelow.

From the above results, in the case of conventional transdermal immunizing method using subcutaneous injection, inflammation in the skin was observed. The degree of inflammation when comparative agent 1 containing FCA was administered was much more severe than the degree when comparative agent 2 without FCA was administered.

## Claims

1. An S/O type transdermal immunizing agent,
comprising an antigen-surfactant complex and an oil phase;
wherein the antigen is covered with the surfactant in the complex;
the complex is in a solid state; and
the complex is dissolved or dispersed in the oil phase.

2. The S/O type transdermal immunizing agent according to claim 1, wherein the antigen is a protein or peptide.

3. The S/O type transdermal immunizing agent according to claim 1, wherein the antigen is an unchanged or inactivated bacterium and/or virus.

4. The S/O type transdermal immunizing agent according to claim 1, wherein the antigen is derived from an animal or plant.

5. The S/O type transdermal immunizing agent according to any one of claims 1 to 4, wherein the surfactant is a nonionic surfactant.

6. The S/O type transdermal immunizing agent according to any one of claims 1 to 5, wherein a HLB value of the surfactant is not more than 10.

7. The S/O type transdermal immunizing agent according to any one of claims 1 to 6, wherein the oil phase is selected from a group consisting of a plant oil, an animal oil, a neutral lipid and a long-chain fatty acid ester.

8. An S/O/W type transdermal immunizing agent, wherein the S/O type transdermal immunizing agent according to any one of claims 1 to 7 is dispersed in an aqueous phase.
